# EUROPEAN PATENT APPLICATION

(11) **EP 4 342 931 A1**
(43) Date of publication of application: **27.03.2024**
(21) Application number: 22804310.5
(22) Date of filing: 07.03.2022
(51) Int. Cl.: C08G 73/06, C07D 307/88

(54) **ACID DIANHYDRIDE**

(30) Priority: 19.05.2021 JP 2021084717
(71) Applicant: MITSUBISHI GAS CHEMICAL COMPANY, INC., Chiyoda-ku Tokyo 100-8324 (JP)
(72) Inventor: WAKAHARA, Daiki, Tokyo 100-8324 (JP); HIKICHI, Tatsuya, Tokyo 100-8324 (JP); OHNISHI, Nobuyoshi, Tokyo 125-8601 (JP)
(74) Representative: Hoffmann Eitle
(86) International application number: PCT/JP2022/009636
(87) International publication number: WO 2022/244390

(57) **Abstract**

An acid dianhydride represented by the following formula (1): 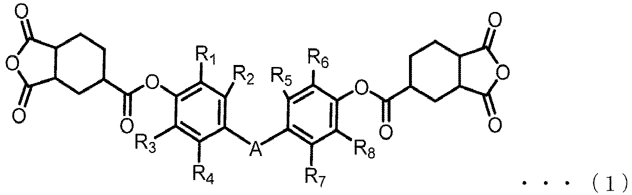 wherein R₁ to R₈ each independently represent a hydrogen atom or an organic group having 1 to 10 carbon atoms; and A represents the following structure.

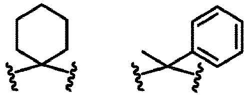

## Description

### Technical Field

The present invention relates to a novel acid dianhydride; and polyamic acid and polyimide derived from the acid dianhydride; and a resin composition containing any of those.

### Background Art

Because of excellent properties such as mechanical properties, chemical resistance, flame retardancy, and electrical properties in addition to heat resistance, polyimides are widely used in various fields as molding materials, composite materials, and electrical and electronic components.

In recent years, a traction motor has been made smaller in size, lighter in weight, and higher in voltage to extend the cruising range of an electric vehicle. Windings whose conductor surfaces are coated with an insulation coating are used in the traction motor. When an electric current flows through the traction motor, a potential difference is generated between these windings or between the windings and the core. As the voltage of the traction motor increases, this potential difference tends to cause partial discharge. The occurrence of such partial discharge causes damage to the insulation coating, and if the damage progresses, a dielectric breakdown occurs.

In order to prevent such partial discharge, it is considered to increase the thickness of an insulating resin. However, the thicker the insulation coating, the smaller the volume fraction of the conductor. Accordingly, the traction motor may need to increase in size or have high electrical resistance, thus being contrary to compactness and high-power output.

Therefore, Patent Literature 1 discloses that a certain amount of inorganic filler is contained in an insulation coating for the purpose of suppressing erosion due to partial discharge.

### Citation List

### Patent Literature

Patent Literature 1: Japanese Patent Laid-Open No. 2019-128995

### Summary of Invention

### Technical Problem

However, since a method described in Patent Literature 1 is to make an insulating resin less susceptible to erosion when partial discharge occurs, the partial discharge will eventually erode the insulating resin and cause a dielectric breakdown. Therefore, as a fundamental solution to the partial discharge associated with smaller size, lighter weight, and higher voltage of a traction motor, it is desired to develop a low dielectric-constant insulation coating material that does not cause partial discharge.

The present invention has been made in view of the above problem, and an object of the present invention is to provide a novel acid dianhydride having a low dielectric constant; and polyamic acid and polyimide derived from the acid dianhydride; and a resin composition containing any of those.

### Solution to Problem

The present inventors have conducted intensive studies to achieve the above object. As a result, the present inventors have found that the above object can be achieved by using a novel acid dianhydride having a predetermined structure and completed the present invention.

In other words, the present invention is as follows.
[1] An acid dianhydride represented by the following formula (1): 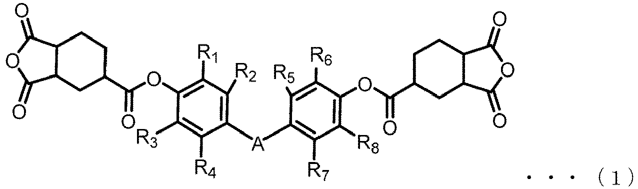 wherein R₁ to R₈ each independently represent a hydrogen atom or an organic group having 1 to 10 carbon atoms; and A represents the following structure. 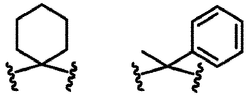
[2] The acid dianhydride according to [1], represented by the following formula (2): 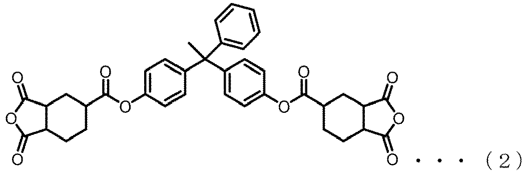
[3] A polyamic acid having a constituent unit represented by the following formula (3): 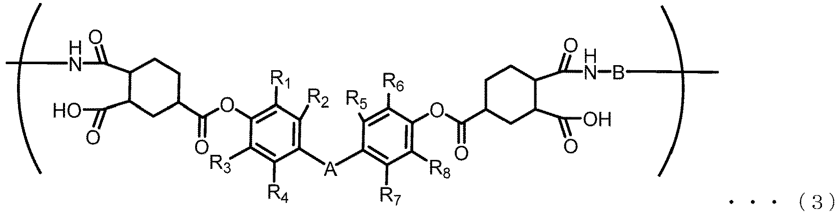 wherein R₁ to R₈ each independently represent a hydrogen atom or an organic group having 1 to 10 carbon atoms; each A independently represents the following structure; and each B independently represents a divalent aromatic group or a aliphatic group. 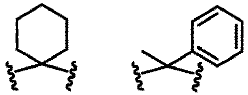
[4] The polyamic acid according to [3], having a constituent unit represented by the following formula (4) : 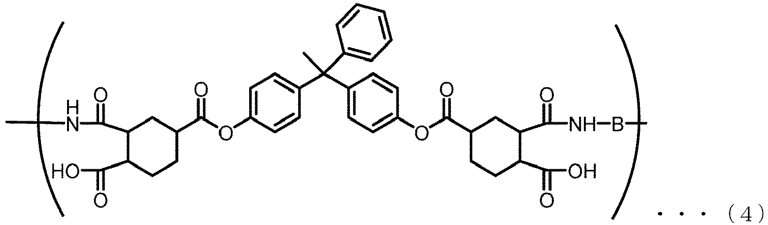 wherein each B independently represents a divalent aromatic group or aliphatic group.
[5] The polyamic acid according to [3] or [4], wherein a content of a constituent unit derived from acid dianhydride represented by the following formula (1) is 10 mol% to 100 mol% based on the total amount of constituent units derived from acid dianhydrides: 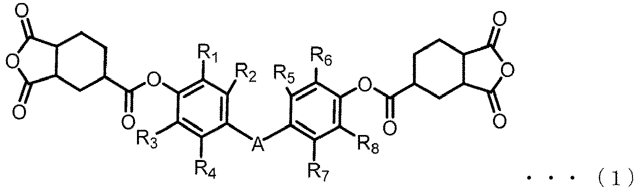 wherein R₁ to R₈ each independently represent a hydrogen atom or an organic group having 1 to 10 carbon atoms; and A represents the following structure. 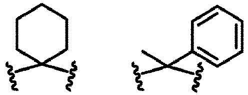
[6] A polyimide having a constituent unit represented by the following formula (5): 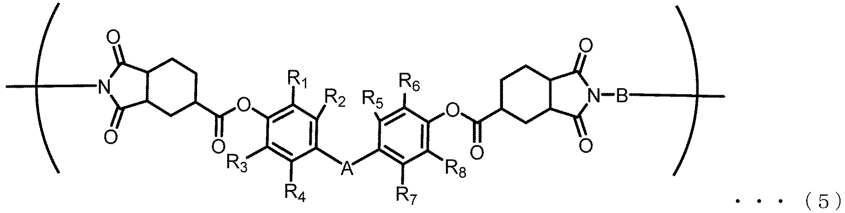 wherein R₁ to R₈ each independently represent a hydrogen atom or an organic group having 1 to 10 carbon atoms; each A independently represents the following structure; and each B independently represents a divalent aromatic group or aliphatic group. 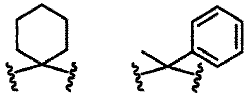
[7] The polyimide according to [6], having a constituent unit represented by the following formula (6): 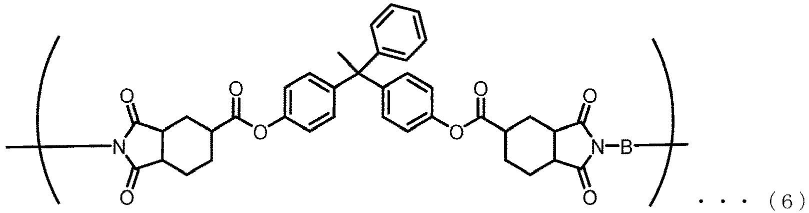 wherein each B independently represents a divalent aromatic group or aliphatic group.
[8] The polyimide according to [6] or [7], wherein a content of a constituent unit derived from acid dianhydride represented by the following formula (1) is 10 mol% to 100 mol% based on the total amount of constituent units derived from acid dianhydrides: 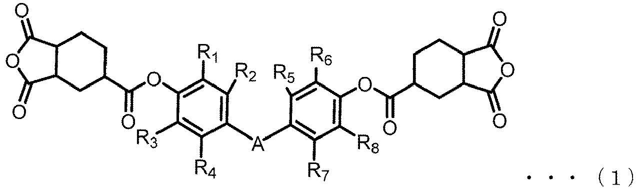 wherein R₁ to R₈ each independently represent a hydrogen atom or an organic group having 1 to 10 carbon atoms; and A represents the following structure. 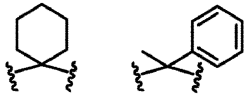
[9] A resin composition comprising the acid dianhydride according to [1] or [2], the polyamic acid according to any one of [3] to [5], or the polyimide according to any one of [6] to [8].

### Advantageous Effect of Invention

The present invention can provide a novel acid dianhydride having a low dielectric constant; and polyamic acid and polyimide derived from the acid dianhydride; and a resin composition containing any of those.

### Description of Embodiments

Hereinafter, an embodiment of the present invention (hereinafter referred to as "the present embodiment") will be described in detail, but the present invention is not limited thereto. Various modifications can be made without departing from the gist of the present invention.

### 1. Acid dianhydride

The acid dianhydride represented by the following formula (1): 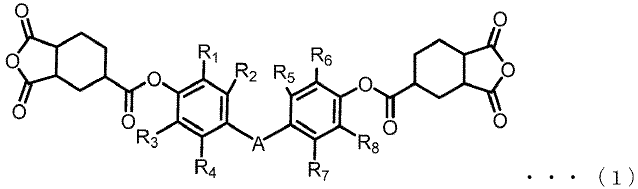 wherein R₁ to R₈ each independently represent a hydrogen atom or an organic group having 1 to 10 carbon atoms; and each A represents the following structure.

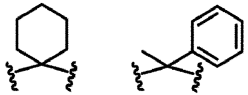

In general formula (1), the organic groups having 1 to 10 carbon atoms represented by R₁ to R₈ are not particularly limited, and examples thereof include alkyl groups such as a methyl group, an ethyl group, an n-propyl group, an isopropyl group, an n-butyl group, an isobutyl group, a sec-butyl group, and a t-butyl group; cycloalkyl groups such as a cyclopropyl group, a cyclobutyl group, a cyclopentyl group, a cyclohexyl group, and a cyclooctyl group; aryl groups such as a phenyl group; aralkyl groups such as a methylphenyl group; and alkenyl groups such as a vinyl group, a propynyl group, and a butynyl group. Among them, alkyl and cycloalkyl groups are preferred. The number of carbon atoms of the organic group is 1 to 10, preferably 1 to 6, and more preferably 1 to 4.

Among them, an acid dianhydride represented by the following formula (2) is preferred. The acid dianhydride thereby tends to have a lower dielectric constant.

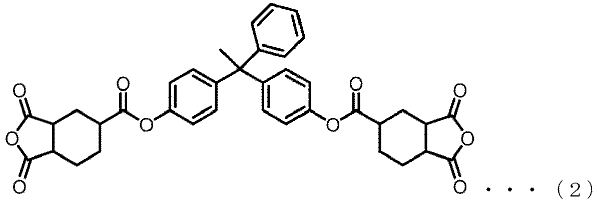

A method for producing the acid dianhydride is not particularly limited, and examples thereof include a method of reacting nuclear hydrogenated trimellitic anhydride chloride with the 4,4'-cyclohexylidenebisphenol or 4,4'-(α-methylbenzylidene)bisphenol.

### 2. Polyamic acid

The polyamic acid of the present embodiment has a constituent unit represented by the following formula (3) : 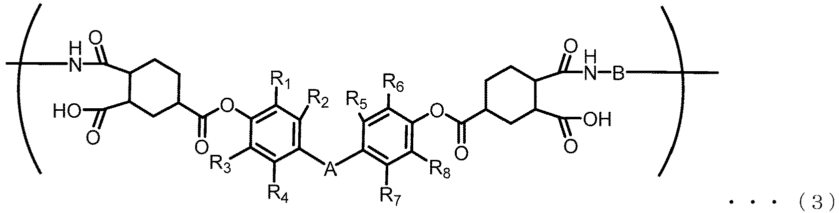 wherein R₁ to R₈ each independently represent a hydrogen atom or an organic group having 1 to 10 carbon atoms; each A independently represents the following structure; and each B independently represents a divalent aromatic group or aliphatic group.

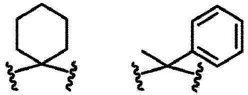

In general formula (3), the organic groups having 1 to 10 carbon atoms represented by R₁ and R₈ are not particularly limited, and examples thereof include those similar to the groups exemplified in general formula (1).

A polyamic acid represented by the following formula (4) is preferred. The polyamic acid thereby tends to have a lower dielectric constant: 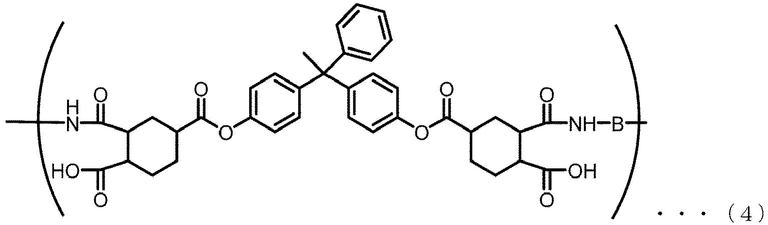 wherein each B independently represents a divalent aromatic group or aliphatic group.

Each B in general formulae (3) and (4) independently represents a divalent aromatic group or a divalent aliphatic group. Such divalent aromatic groups are not particularly limited, and examples thereof include divalent monocyclic aromatic groups such as 1,3-phenylene, 1,4-phenylene, 4,4'-biphenylene, 5-chloro-1,3-phenylene, and 5-methoxy-1,3-phenylene; divalent fused-polycyclic aromatic groups such as 1,4-naphthylene, 2,6-naphthylene, 1,4-anthrylene, 9,10-anthrylene, and 3,4-perylenylene; and divalent non-fused polycyclic aromatic groups in which monocyclic aromatic groups such as phenyl and biphenyl, including 2,2-propylidenebis(1,4-phenylene), 2,2-(1,1,1,3,3,3-hexafluoropropylidene)bis(1,4-phenylene), carbonylbis(1,4-phenylene), oxybis(1,4-phenylene), sulfonylbis(1,4-phenylene), and 9,9-fluorenylidenebis(1,4-phenylene) are mutually connected via an oxygen atom, a carbonyl group, an ester group, and a linking group such as methylene, ethylidene, 1-methylethylidene, 1,1-propylidene, 1,4-phenylenebis(1-methylethylidene), 1,3-phenylenebis(1-methylethylidene), cyclohexylidene, phenylmethylene, naphthylmethylene, and 1-phenylethylidene. The aromatic groups may have a substituent such as a methyl group or an ethyl group.

The divalent aliphatic groups are not particularly limited, and examples thereof include divalent linear aliphatic groups such as a methylene group, an ethylene group, a propylene group, a butylene group, a pentylene group, a hexylene group, an octylene group, a decylene group, a dodecylene group, a hexadecylene group, and an octadecylene group; divalent branched aliphatic groups such as an isopropylene group, an isobutylene group, a tertiary butylene group, a neopentylene group, a 2-hexylene group, a 2-octylene group, a 2-decylene group, a 2-dodecylene group, a 2-hexadecylene group, and a 2-octadecylene group; and divalent cyclic aliphatic groups such as a cyclopropylene group, a cyclobutylene group, a cyclopentylene group, a cyclohexylene group, a cyclooctylene group, a cyclodecylene group, a cyclododecylene group, a cyclohexadecylene group, and a cyclooctadecylene group.

The polyamic acid of the present embodiment may be a homopolymer of an amic acid represented by general formula (3) or a copolymer having a constituent unit other than the constituent unit derived from acid dianhydride represented by general formula (1) as a constituent unit derived from acid dianhydride. In this case, the polyamic acid of the present embodiment may be a block copolymer of a constituent unit of the amic acid represented by general formula (3) and a constituent unit of another amic acid or a random copolymer.

In the polyamic acid of the present embodiment, the constituent unit derived from acid dianhydride represented by general formula (1) refers to a residue formed when a diamine reacts with an acid anhydride group in general formula (1).

In the polyamic acid of the present embodiment, the content of the constituent unit derived from acid dianhydride represented by general formula (1) is preferably 10 to 100 mol%, more preferably 30 to 100 mol%, still more preferably 50 to 100 mol%, further preferably 70 to 100 mol%, even more preferably 80 to 100 mol%, and particularly preferably 90 to 100 mol% based on the total amount of constituent units derived from acid dianhydrides. When the content of the constituent unit derived from acid dianhydride represented by general formula (1) is within the above range, the polyamic acid tends to have a lower dielectric constant.

Other acid dianhydrides constituting constituent units other than the constituent unit represented by general formula (3) are not particularly limited, and examples thereof include pyromellitic dianhydride, 1,2,3,4-benzenetetracarboxylic dianhydride, 3,3',4,4'-benzophenonetetracarboxylic dianhydride, 2,2',3,3'-benzophenonetetracarboxylic dianhydride, 3,3',4,4'-biphenyltetracarboxylic dianhydride, 2,2',3,3'-biphenyltetracarboxylic dianhydride, 2,3,3',4'-biphenyltetracarboxylic dianhydride, 2,2-bis(3,4-dicarboxyphenyl)propane dianhydride, 2,2-bis(2,3-dicarboxyphenyl)propane dianhydride, bis(3,4-dicarboxyphenyl)ether dianhydride, bis(2,3-dicarboxyphenyl)ether dianhydride, bis(3,4-dicarboxyphenyl)sulfone dianhydride, bis(2,3-dicarboxyphenyl)sulfone dianhydride, 2,3-bis(3,4-dicarboxyphenyl)-1,1,1,3,3,3-hexafluoropropane dianhydride, 2,2-bis(2,3-dicarboxyphenyl)-1,1,1,3,3,3-hexafluoropropane dianhydride, 9,9-bis(4-(3,4-dicarboxyphenoxy)phenyl)fluorene dianhydride, 9,9-bis(4-(2,3-dicarboxyphenoxy)phenyl)fluorene dianhydride, 2,3,6,7-naphthalenetetracarboxylic dianhydride, 1,4,5,8-naphthalenetetracarboxylic dianhydride, and 3,4,9,10-perylenetetracarboxylic dianhydride.

The polyamic acid of the present embodiment preferably has a weight average molecular weight of 3000 to 150000, more preferably 5000 to 125000, and still more preferably 10000 to 100000.

A method for producing the polyamic acid of the present embodiment is not particularly limited, and examples thereof include a polycondensation method of the acid dianhydride represented by general formula (1) with diamines. In this process, the other acid dianhydrides may be used in combination as necessary. The diamines may be used alone or in combination of two or more.

Examples of the diamines include diamines having a divalent aromatic group or a divalent aliphatic group in B above. Such diamines are not particularly limited, and examples thereof include p-phenylenediamine, m-phenylenediamine, 3,4'-diaminodiphenyl ether, 4,4'-diaminodiphenyl ether, 1,5-naphthylenediamine, 4,4'-diaminodiphenylmethane, 3,4'-diaminodiphenyl sulfone, 4,4'-diaminodiphenyl sulfone, 2,4- diaminochlorobenzene, 2,2-bis[4-(4-aminophenoxy)phenyl]propane, 2,2-[4-(4-aminophenoxyphenyl)][4-(3-aminophenoxyphenyl)]propane, 2,2-bis[3-(3-aminophenoxy)phenyl]-1,1,1,3,3,3-hexafluoropropane, 2,2-bis[4-(4-aminophenoxy)phenyl]-1,1,1,3,3,3-hexafluoropropane, 2,2-[4-(4-aminophenoxyphenyl)][4-(3-aminophenoxyphenyl)]-1,1,1,3,3,3-hexafluoropropane, 1,3-bis(3-aminophenoxy)benzene, 1,4-bis(3-aminophenoxy)benzene, 1,4-bis(4-aminophenoxy)benzene, 1,3-bis(4-aminophenoxy)benzene, and 4,4'-bis(4-aminophenoxy) biphenyl.

Polycondensation of a diamine with acid dianhydride can be carried out in an organic solvent. The organic solvent used in the polycondensation is not particularly limited, and examples thereof may include N,N-dimethylformamide, N,N-dimethylacetamide, N,N-dimethylmethoxyacetamide, N,N-diethylmethoxyacetamide, N-methyl-2-pyrrolidone, N-methylcaprolactam, 1,2-dimethoxyethane, diethylene glycol dimethyl ether, diethylene glycol ethyl methyl ether, diethylene glycol diethyl ether, tetrahydrofuran, 1,3-dioxane, 1,4-dioxane, pyridine, picoline, dimethyl sulfoxide, dimethyl sulfone, and tetramethylurea. These organic solvents may be used alone or in a mixture of two or more.

### 3. Polyimide

The polyimide of the present embodiment has a constituent unit represented by the following formula (5) : 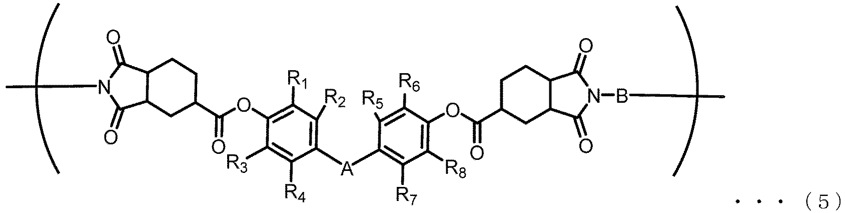 wherein R₁ to R₈ each independently represent a hydrogen atom or an organic group having 1 to 10 carbon atoms; each A independently represents the following structure; and each B independently represents a divalent aromatic group or a aliphatic group.

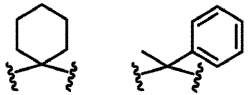

In general formula (5), the organic groups having 1 to 10 carbon atoms represented by R₁ to R₈ are not particularly limited, and examples thereof include those similar to the groups exemplified in general formula (1). Furthermore, in general formula (5), B is not particularly limited, and examples thereof include those similar to the groups exemplified in general formula (3).

A polyimide represented by the following formula (6) is preferred. The polyimide thereby tends to have a lower dielectric constant: 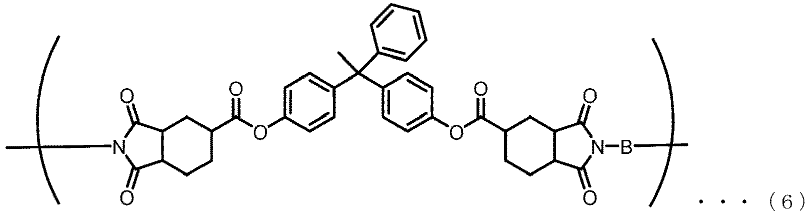 wherein each B independently represents a divalent aromatic group or aliphatic group.

The polyimide of the present embodiment may be a homopolymer of the polyimide represented by general formula (5) or a copolymer having a constituent unit other than the constituent unit derived from acid dianhydride represented by general formula (1) as a constituent unit derived from acid dianhydride. In this case, the polyimide may be a block copolymer of a constituent unit of the polyimide represented by general formula (5) and a constituent unit of another polyimide or a random copolymer.

In the polyimide of the present embodiment, the constituent unit derived from acid dianhydride represented by general formula (1) refers to a constituent unit obtained by imidizing a residue formed by reaction of a diamine with an acid anhydride group in general formula (1) by cyclodehydration.

In the polyimide of the present embodiment, the content of the constituent unit derived from acid dianhydride represented by general formula (1) is preferably 10 to 100 mol%, more preferably 30 to 100 mol%, still more preferably 50 to 100 mol%, further preferably 70 to 100 mol%, even more preferably 80 to 100 mol%, and particularly preferably 90 to 100 mol% based on the total amount of constituent units derived from acid dianhydrides. When the content of the constituent unit derived from acid dianhydride represented by general formula (1) is within the above range, the polyimide tends to have a lower dielectric constant.

A method for producing the polyimide of the present embodiment is not particularly limited, and examples thereof include a method of imidizing the polyamic acid by cyclodehydration.

Examples of the imidization methods include thermal imidization methods and chemical imidization methods. Examples of thermal imidization methods include a method (a) in which a polyamic acid solution is cast on a smooth surface substrate such as glass or metal and then heated for cyclodehydration and a method (b) in which a polyamic acid solution is directly heated for cyclodehydration. Examples of solvents for the polyamic acid solution in these methods include organic solvents similar to those used in the production of polyamic acid.

In the thermal imidization method (a), a film polyimide can be obtained by heating a thin film formed by casting a polyamic acid solution on a substrate under normal or reduced pressure. In this case, the heating temperature for cyclodehydration is usually 100 to 400°C, preferably 150 to 350°C, and it is preferable to gradually raise the temperature during the reaction.

In the thermal imidization method (b), the polyimide is obtained as a powder or solution by heating the polyamic acid solution. In this case, the heating temperature for cyclodehydration is usually 80 to 300°C, preferably 100 to 250°C.

In the thermal imidization method (b), in order to facilitate the removal of by-product water, it is also possible to allow the presence of components that are azeotropic with water and particularly easily separated from water outside the reaction system, for example, aromatic hydrocarbons such as benzene, toluene, and xylene as dehydrating agents. In the thermal imidization method (b), furthermore, catalysts such as tertiary amines, for example, aliphatic tertiary amines such as trimethylamine, triethylamine, tri-n-propylamine, tri-i-propylamine, and tri-n-butylamine; aromatic tertiary amines such as N,N-dimethylaniline and N,N-diethylaniline; and heterocyclic tertiary amines such as pyridine, quinoline, and isoquinoline may be used to promote cyclodehydration.

Examples of the chemical imidization methods include a method (c) of polyimidization in a solution state using cyclization agents that dehydrate and cyclize polyamic acid, whereby the polyimide can be obtained as a powder or solution.

Examples of the solvents used in this method include organic solvents similar to those used in the production of polyamic acid. The cyclization agents used in the chemical imidization method (c) are not particularly limited, and examples thereof include acid anhydrides such as acetic anhydride, propionic anhydride, and butyric anhydride. These cyclization agents may be used alone or in a mixture of two or more.

The reaction temperature in the chemical imidization method (c) is usually 0 to 200°C. Note that in the chemical imidization method, tertiary amines can be used as a catalyst as in the case of the thermal imidization method.

When the polyimide is obtained as a powder by a thermal imidization method or a chemical imidization method, the polyimide powder can be separated and recovered from a medium by an appropriate method such as filtration, spray drying, and steam distillation. The imidization rate of the polyimide of the present embodiment is 50% or more, preferably 90% or more.

### 4. Resin composition

The resin composition of the present embodiment contains the above acid dianhydride, the above polyamic acid, or the above polyimide and may also contain other resins and inorganic fillers as necessary.

The resin-molded product of the present embodiment has a low dielectric constant and can thus be suitably used as a coating material for electric wires used, for example, in driving motors, generators, and accessory motors of hybrid or electric vehicles. In addition, the product can also be suitably used as a protective film for high-frequency and/or high-voltage electric wires, or an insulating film for circuit boards.

### Examples

Hereinafter, the present invention will be described in more detail with reference to Examples and Comparative Examples. However, the present invention is not limited at all by the following Examples.

### [NMR measurement]

1H-NMR was measured by dissolving measurement samples in heavy dimethyl sulfoxide (DMSO-d6; deuteration ratio of D99.9%, 0.05 vol% TMS) containing tetramethylsilane as an internal standard. ASCEND TM 500 manufactured by BRUKER Corporation was used as an NMR instrument.

### [Dielectric constant]

The dielectric constant at 10 GHz of polyimide films obtained in Examples 3 and 4, and Comparative Example 1 was measured three times by a cavity resonator perturbation method (Agilent 8722 ES, manufactured by Agilent Technologies, Inc.) to determine the average value.

### [Weight average molecular weight]

The weight average molecular weight of polyamic acids obtained in Examples 3 and 4 in terms of polymethyl methacrylate was measured under the following measurement conditions.

### (Measurement conditions)

Device: CBM-20A, SIL-10ADvp, LC-10ADvp, DGU-12A, SPD-10Avp, CTO-10Avp, RID-10A, FRC-10A (all manufactured by Shimazu Corporation)
Column: Shodex GPC K-804
Column temperature: 50°C
Mobile phase: N-methylpyrrolidone (LiBr (30 mM), H₃PO₄ (30 mM))
Mobile phase flow rate: 0.7 mL/min
Molecular weight standard: polymethyl methacrylate (Shodex M-75)

### [Example 1]

In a 100 mL three-necked flask, 27.3 g of nuclear hydrogenated trimellitic anhydride chloride and 120 mL of super dehydrated stabilizer-free THF were dissolved under nitrogen flow (Solution A). To a 200 mL two-necked flask purged with nitrogen, 16.1 g of 4,4'-cyclohexylidenebisphenol, 69 mL of super dehydrated stabilizer-free THF, and 23 mL of pyridine were added and stirred at room temperature (Solution B). Solution B was slowly added to Solution A in an ice bath, and the mixture was then stirred at room temperature for 24 hours. After stirring was completed, 200 mL each of THF and saturated saline was put in a beaker, and the reaction liquid was poured into the beaker and stirred for several minutes. This was transferred to a separating funnel to separate an aqueous layer. The mixture was further washed three times with saturated brine, and a THF layer was then collected from the separating funnel and dried over magnesium sulfate. After removal of the drying agent by gravity filtration, the solvents were evaporated with an evaporator to obtain a solid.

Acetic anhydride was added in an amount twice the obtained solid, and the mixture was heated and stirred in an oil bath at 85°C for 2 hours to subject the solid to cyclization treatment. Then, the mixture was allowed to cool and stand overnight. After standing, the mixture was poured into isopropyl ether in an amount 10 times the solid, and the mixture was stirred to precipitate a solid. The precipitate was filtered through Kiriyama funnel filter paper 5C, and the filtrate was dried in vacuum to yield 26.5 g of white powder (yield: 69.0%). The structural formula and 1H-NMR results of the resulting compound are shown below. 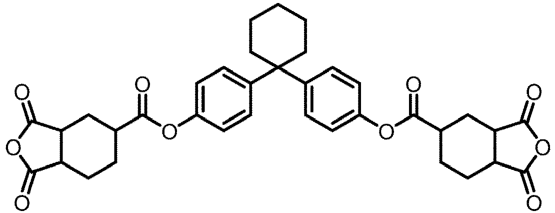 1H-NMR (500 MHz, DMSO-d6):
δ 7.34-7.32 (d, 4H), δ 7.03-7.00 (d, 4H), δ 3.56-3.52 (m, 2H), δ 3.37-3.32 (m,2H), δ 2.76-2.70 (m, 2H), δ 2.33-2.23 (m, 6H), δ 2.07-2.01 (m, 2H), δ 1.97-1.92 (m, 2H), δ 1.82-1.75 (m, 2H), δ 1.73-1.66 (m, 2H), δ 1.50-1.42 (m, 8H)

### [Example 2]

In a 300 mL three-necked flask, 23.4 g of nuclear hydrogenated trimellitic anhydride chloride and 100 mL of super dehydrated stabilizer-free THF were dissolved under nitrogen flow (Solution A). To a 200 mL two-necked flask purged with nitrogen, 14.6 g of 4,4'-methylbenzylidene bisphenol, 60 mL of super dehydrated stabilizer-free THF, and 20 mL of pyridine were added and stirred at room temperature (Solution B). Solution B was slowly added to Solution A in an ice bath, and the mixture was then stirred at room temperature for 24 hours. After stirring was completed, 200 mL each of THF and saturated saline was put in a beaker, and the reaction liquid was poured into the beaker and stirred for several minutes. This was transferred to a separating funnel to separate an aqueous layer. The mixture was further washed three times with saturated brine, and a THF layer was then collected from the separating funnel and dried over magnesium sulfate. After removal of the drying agent by gravity filtration, the solvents were evaporated with an evaporator to obtain a solid.

Acetic anhydride was added in an amount twice the obtained solid, and the mixture was heated and stirred in an oil bath at 85°C for 2 hours to subject the solid to cyclization treatment. Then, the mixture was allowed to cool and stand overnight. After standing, the mixture was poured into isopropyl ether in an amount 10 times the solid, and the mixture was stirred to precipitate a solid. The precipitate was filtered through Kiriyama funnel filter paper 5C, and the filtrate was dried in vacuum to yield 21.85 g of white powder (yield: 67.2%). The structural formula and 1H-NMR results of the resulting compound are shown below. 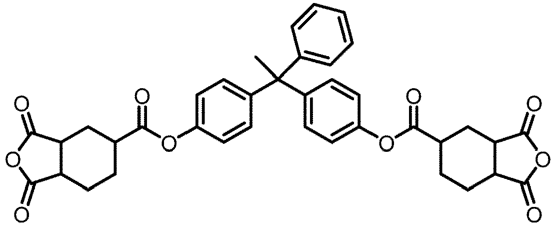 1H-NMR (500MHz, DMSO-d6):
δ 7.32-7.29 (t, 2H), δ 7.24-7.21 (m, 1H), δ 7.09-7.04 (m, 10H), δ 3.57-3.53 (m, 2H), δ 3.39-3.33 (m, 2H), δ 2.78-2.73 (m, 2H), δ 2.36-2.31 (m, 2H), 2.13 (s, 3H), δ 2.07-2.03 (m, 2H), δ 1.99-1.95 (m, 2H), δ 1.84-1.76 (m, 2H), δ 1.75-1.68 (m, 2H), δ 1.53-1.46 (m, 2H)

### [Example 3]

In a 300 mL separable flask, 2.00 g of 4,4'-diaminodiphenyl ether (hereinafter abbreviated as "ODA") and 9.50 g of N-methylpyrrolidone (hereinafter referred as "NMP") sufficiently dehydrated with molecular sieves 4Å were put and dissolved under nitrogen flow. To this solution was added 6.28 g of the acid dianhydride obtained in Example 1 in an ice bath, and 10.0 g of NMP was then added thereto to initiate the polymerization reaction from an initial total solute concentration of 30% by weight. The mixture was stirred with a mechanical stirrer at 200 rpm. After 2 hours and 30 minutes, an increase in viscosity was confirmed, so that NMP was added to dilute the mixture to 25% by weight. After 71 hours from the initiation of polymerization, the solution was diluted to 20% by weight with NMP to obtain a pale yellow polyamic acid. The obtained polyamic acid had a weight average molecular weight of 76,640. The resulting polyamic acid solution was applied onto a base material, dried at 100°C for 1 hour, then heated at 200°C for 1 hour and at 250°C for 30 minutes, and peeled from the base material to obtain a polyimide film. This polyimide had a dielectric constant of 2.56 at 10 GHz.

### [Example 4]

N₂ gas was flowed into a 300 mL separable flask. In the separable flask, 2.00 g of ODA and 9.80 g of NMP sufficiently dehydrated with molecular sieves 4Å were put and dissolved. To this solution was added 6.50 g of the acid dianhydride obtained in Example 2 in an ice bath, and 10.0 g of NMP was then added thereto to initiate the polymerization reaction from an initial total solute concentration of 30% by weight. The mixture was stirred with a mechanical stirrer at 200 rpm and was diluted to 20% by weight with NMP after 63 hours to obtain a pale yellow polyamic acid. The obtained polyamic acid had a weight average molecular weight of 38,827. The resulting polyamic acid solution was applied onto a base material, dried at 100°C for 1 hour, then heated at 200°C for 1 hour and at 250°C for 30 minutes, and peeled from the base material to obtain a polyimide film. This polyimide had a dielectric constant of 2.53 at 10 GHz.

### [Comparative Example 1]

In Comparative Example 1, Kapton (R) (manufactured by DU PONT-TORAY CO., LTD.) was used. This polyimide had a dielectric constant of 2.87 at 10 GHz.

**[Table 1]**

| | Structure | Dielectric constant (10 GHz) |
|---|---|---|
| Example 3 | 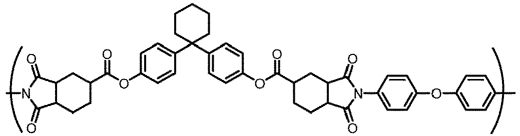 | 2.56 |
| Example 4 | 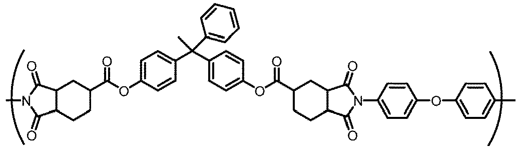 | 2.53 |
| Comparative Example 1 | 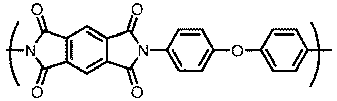 | 2.87 |

### Industrial Applicability

In the present invention, a novel acid dianhydride, a polyamic acid, and a polyimide have industrial applicability as coating materials for electric wires used, for example, in traction motors, generators, accessory motors of hybrid or electric vehicles, or as protective films for other high-frequency and/or high-voltage electric wires.

## Claims

1. An acid dianhydride represented by the following formula (1): 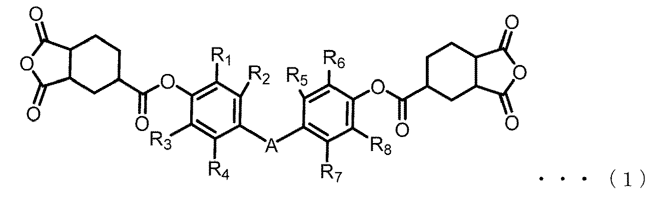 wherein R₁ to R₈ each independently represent a hydrogen atom or an organic group having 1 to 10 carbon atoms; and A represents the following structure.

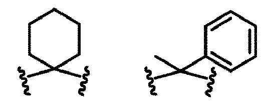

2. The acid dianhydride according to claim 1, represented by the following formula (2):

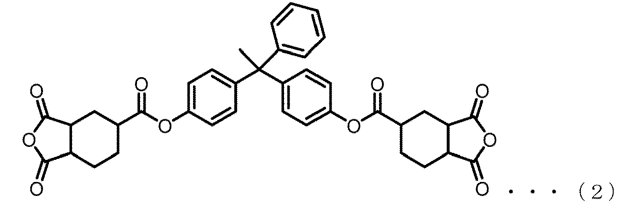

3. A polyamic acid having a constituent unit represented by the following formula (3): 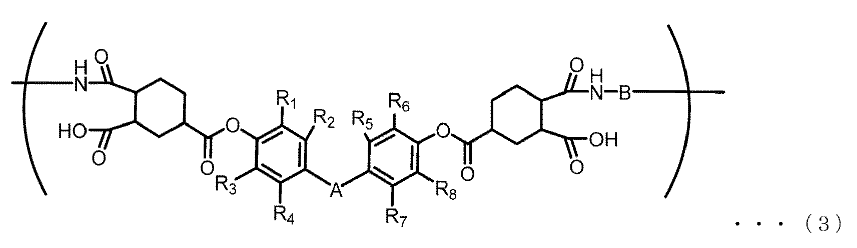 wherein R₁ to R₈ each independently represent a hydrogen atom or an organic group having 1 to 10 carbon atoms; each A independently represents the following structure; and each B independently represents a divalent aromatic 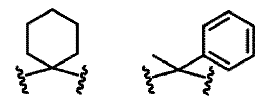 group or aliphatic group.

4. The polyamic acid according to claim 3, having a constituent unit represented by the following formula (4) : 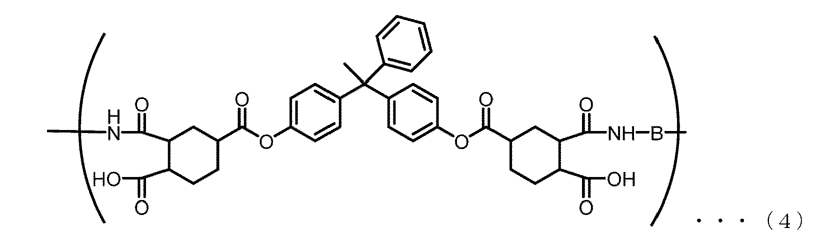 wherein each B independently represents a divalent aromatic group or aliphatic group.

5. The polyamic acid according to claim 3 or 4, wherein a content of a constituent unit derived from acid dianhydride represented by the following formula (1) is 10 mol% to 100 mol% based on the total amount of constituent units derived from acid dianhydrides: 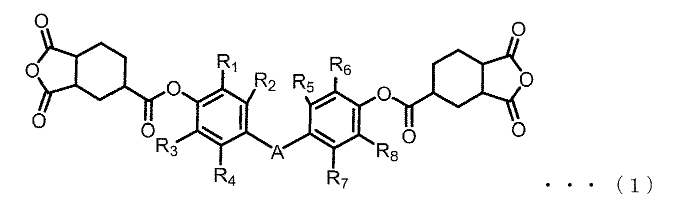 wherein R₁ to R₈ each independently represent a hydrogen atom or an organic group having 1 to 10 carbon atoms; and A represents the following structure.

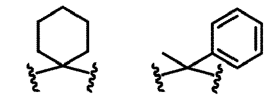

6. A polyimide having a constituent unit represented by the following formula (5): 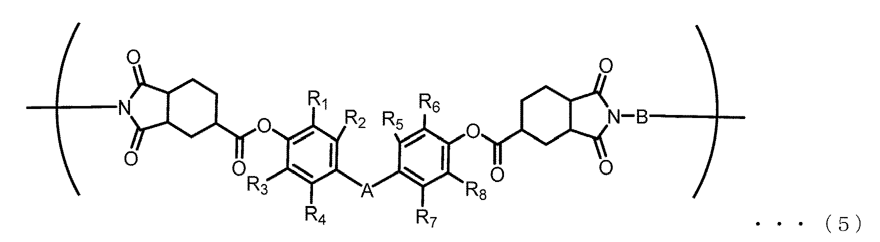 wherein R₁ to R₈ each independently represent a hydrogen atom or an organic group having 1 to 10 carbon atoms; each A independently represents the following structure; and each B independently represents a divalent aromatic group or aliphatic group.

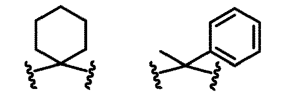

7. The polyimide according to claim 6, having a constituent unit represented by the following formula (6) : 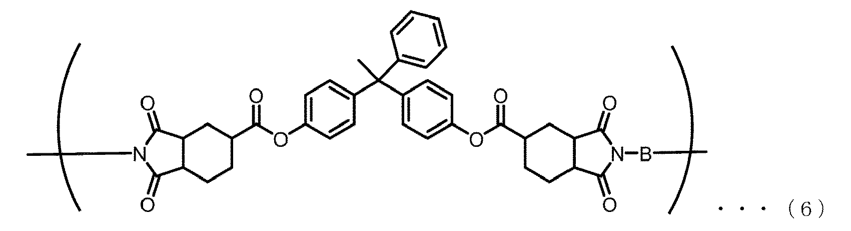 wherein each B independently represents a divalent aromatic group or aliphatic group.

8. The polyimide according to claim 6 or 7, wherein a content of a constituent unit derived from acid dianhydride represented by the following formula (1) is 10 mol% to 100 mol% based on the total amount of constituent units derived from acid dianhydrides: 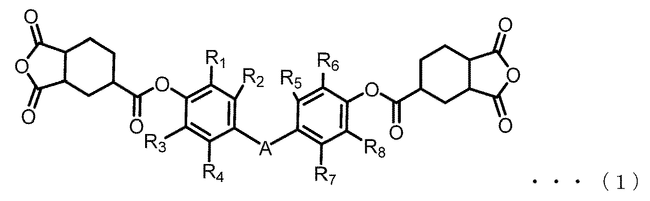 wherein R₁ to R₈ each independently represent a hydrogen atom or an organic group having 1 to 10 carbon atoms; and A represents the following structure.

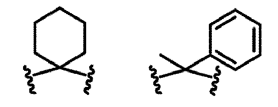

9. A resin composition comprising the acid dianhydride according to claim 1 or 2, the polyamic acid according to any one of claims 3 to 5, or the polyimide according to any one of claims 6 to 8.
